# EUROPEAN PATENT APPLICATION

(11) **EP 4 585 210 A1**
(43) Date of publication of application: **16.07.2025**
(21) Application number: 23870971.1
(22) Date of filing: 27.09.2023
(51) Int. Cl.: A61K 9/20, A61K 9/52, A61K 47/34, A61K 47/02, A61K 47/12, A61K 31/519, A61K 31/485, A61P 25/36

(54) **COMPOUND SLOW-RELEASE IMPLANT OF NALTREXONE AND RISPERIDONE, PREPARATION METHOD THEREFOR, AND USE THEREOF**

(30) Priority: 28.09.2022 CN 202211195550
(71) Applicant: Shenzhen Sciencare Pharmaceutical Co., Ltd., Shenzhen, Guangdong 518118 (CN)
(72) Inventor: YAN, Xieguo, Shenzhen, Guangdong 518000 (CN); YAN, Fuqiao, Shenzhen, Guangdong 518000 (CN); HUANG, Yamin, Shenzhen, Guangdong 518000 (CN); YIN, Shugui, Shenzhen, Guangdong 518000 (CN); QU, Wei, Shenzhen, Guangdong 518000 (CN); ZHANG, Tao, Shenzhen, Guangdong 518000 (CN); DENG, Xingjian, Shenzhen, Guangdong 518000 (CN); QIU, Xinmin, Shenzhen, Guangdong 518000 (CN)
(74) Representative: karo IP
(86) International application number: PCT/CN2023/122253
(87) International publication number: WO 2024/067745

(57) **Abstract**

Disclosed is a compound sustained release implant of naltrexone and risperidone, and a preparation method and application thereof, which belong to the technical field of implant preparation. The naltrexone compound sustained-release implant includes naltrexone, risperidone, a degradable material A, a degradable material B, an additive and a lubricant. By controlling a mass ratio of naltrexone to risperidone to be (20-30):1, a weight-average molecular weight of the degradable material A to be 10-15W, and a weight-average molecular weight of the degradable material B in risperidone microspheres to be 2W-4W, the compound sustained release implant of naltrexone and risperidone can not only effectively inhibit amphetamine type drug relapse and reduce side effects of risperidone, but also realize the synchronous release of naltrexone and risperidone.

## Description

### Field of the Invention

The present application relates to the technical field of implant preparation, in particular to a compound sustained release implant of naltrexone and risperidone, and a preparation method and application thereof.

### Background of the Invention

Many drugs in clinical have a short half life in vivo and are very unstable. When these drugs are administered in conventional preparations, they stay in the body for a short time, and should be administered frequently, which is inconvenient to use. Moreover, plasma drug concentration may have an obvious peak-trough effect, resulting in serious adverse reactions and even repeated attacks of the disease. Therefore, in order to effectively treat the diseases, it is necessary to provide an appropriate drug delivery system to keep the plasma drug concentration in the body relatively stable for a long time after administration, and to keep it within a range between the minimum therapeutic level and the toxic level, so as to improve the efficacy and safety of the drugs, and reduce the total dose of the drugs, so that the minimum dose can achieve the maximum efficacy.

An implant is a kind of sterile solid preparation made of drugs and excipients for implanting into the body. An implant is usually implanted in the body by using a special syringe, or can be implanted by a surgical incision. The administered drug is absorbed via subcutaneous or mucosal routes and directly enters the systemic circulation to exert its therapeutic effects, so the first pass effect is avoided, the bioavailability is improved, and the action time is longer, even up to several years.

Naltrxone is a pure opioid receptor antagonist and has a blocking effect on µ-, δ*-,* κ-opioid receptors. It can block drug relapse, thus weaken the positive reinforcement and negative reinforcement, and play a good role in preventing relapse. It has been reported that naltrexone has shown clinical effect in new drug treatment, which has been paid attention to by scientists. Its chemical structure is as follows:

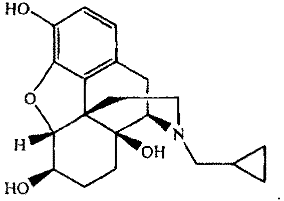

Amphetamine-type stimulants raise dopamine level in synaptic cleft, and also raise 5-hydroxytryptamine level. Risperidone has a good effect on positive and negative symptoms and accompanying affective symptoms (such as anxiety, depression, etc.), and can reduce the affective symptoms associated with schizophrenia. Based on this, risperidone alleviates psychiatric symptoms of amphetamine-type stimulant addicts, which can reduce administration frequency and craving of drugs, and is well tolerated. Its chemical structure is as follows:

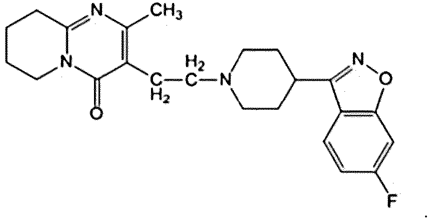

Amphetamine-type stimulant addicts will become physically and mentally dependent after detoxification, and if they are not treated or controlled in time, they are very likely to relapse. The compliance of patients with drugs is a challenging problem.

Chinese patent CN112245434A discloses a compound sustained release composition of naltrexone and risperidone, which is used to prepare a sustained release preparation for preventing relapse of new drugs, where a ratio of naltrexone: risperidone = 5:1-10:1. Although the sustained release preparation prepared in such a ratio can achieve the prevention of relapse of new drugs, it is found that there are relatively serious extrapyramidal symptoms, and amenorrhea, galactorrhea, and sexual dysfunction caused by increased prolactin levels, showing somnolence, dizziness, nausea, and skin rash. In addition, in order to achieve the synchronous release of naltrexone and risperidone, a large amount of magnesium stearate is added to the prescription. There is a burst effect of magnesium stearate during release, with a risk of exceeding the release limit of magnesium ion Mg²⁺ (according to the information of the inactive ingredients database in the United States, no more than 0.5 mg per day), and the safety of the preparation needs to be improved. In addition, the spray coating of polymer solution with high viscosity has poor process operability.

Therefore, it is necessary to develop a compound sustained release implant with fewer excipients and the ability to achieve synchronous release of naltrexone and risperidone.

### Summary of the Invention

Based on the shortcomings of the prior art, the present application aims to provide a compound sustained release implant with fewer excipients and the ability to achieve synchronous release of naltrexone and risperidone, and preparation method and application thereof.

To achieve the above purposes, the following technical solutions are adopted in the present application:
A naltrexone compound sustained-release implant, including the following components: naltrexone, risperidone, a degradable material A, a degradable material B, an additive and a lubricant.

A mass ratio of naltrexone to risperidone is (20-30) : 1.

A mass ratio of naltrexone to the degradable material A is (10-40) : (60-90).

A mass ratio of risperidone to the degradable material B is (40-60) : (40-60).

The degradable material A has a weight-average molecular weight of 10W-15W.

The degradable material B has a weight-average molecular weight of 2W-4W.

The degradable material A is selected from one or more of the following: PEG-PLGA, PLA, PLGA and PCL.

The degradable material B is selected from one or more of the following: PLA, PLGA and PCL.

The additive is selected from one or more of the following: sodium chloride, sodium bicarbonate, disodium hydrogencarbonate, potassium chloride and phosphate; preferably sodium chloride.

The lubricant is selected from one or more of the following: magnesium stearate, stearic acid and sodium stearyl fumarate; preferably magnesium stearate.

A preparation method of the naltrexone compound sustained-release implant, including the following steps:
(1) preparation of naltrexone microspheres:
   (1.1) dissolving an additive in water, to obtain an aqueous phase W₁;
   (1.2) dissolving naltrexone and 30% -60% of a degradable material A in an organic solvent A, to obtain an oil phase O₁;
   (1.3) adding the aqueous phase W₁ into the oil phase O₁, ultrasonically emulsifying to form a primary emulsion W₁/O₁;
   (1.4) dissolving the remaining degradable material A in the organic solvent A, to obtain an oil phase O₂;
   (1.5) adding the W₁/O₁ primary emulsion into the oil phase O₂, ultrasonically emulsifying to form W₁/O₁/O₂ multiple emulsion;
   (1.6) preparing an aqueous solution of polyvinyl alcohol (PVA) to obtain an aqueous phase W₂;
   (1.7) dispersing the W₁/O₁/O₂ multiple emulsion to the aqueous phase W₂, stirring until the organic solvent A is volatilized, filtering and collecting resulting microspheres, washing and drying to obtain the naltrexone microspheres;
(2) preparation of risperidone microspheres:
   (2.1) dissolving risperidone and 50%-80% of a degradable material B in the organic solvent A, to obtain an oil phase O;
   (2.2) preparing an aqueous solution of PVA to obtain an aqueous phase W;
   (2.3) adding the oil phase O into the aqueous phase W, stirring until the organic solvent A is volatilized, filtering and collecting resulting microspheres, washing and drying to obtain the risperidone microspheres;
(3) preparation of polylactic acid (PLA) blank particles:
   dissolving the remaining degradable material B in an organic solvent B to obtain a solution; adding the solution dropwise into a phosphate buffer to obtain polymer blank particles;
(4) blending and tableting
   blending the naltrexone microspheres and the risperidone microspheres, adding and blending with the polymer blank particles and a lubricant, then pressing into cylindrical tablets;
(5) irradiation sterilization
   sterilizing the pressed cylindrical tablets by irradiation to obtain the naltrexone compound sustained-release implant.

In step (1.1), a concentration of the additive in the aqueous phase W₁ is 0-30 mg/mL; preferably 10-25 mg/mL.

In steps (1.2), (1.4), (1.7), (2.1) and (2.3), the organic solvent A is selected from dichloromethane or/and ethyl acetate; preferably dichloromethane.

In step (1.2), a concentration of naltrexone in the oil phase O₁ is 100-300 mg/mL; preferably 150-250 mg/mL.

In step (1.2), a concentration of the degradable material A in the oil phase O₁ is 100-300 mg/mL; preferably 100-200 mg/mL.

In step (1.4), a concentration of the degradable material A in the oil phase O₂ is 200-400 mg/mL; preferably 250-350 mg/mL.

In step (1.6), a mass fraction of PVA in the aqueous phase W₂ is 0.1-1.2%.

In some preferred embodiments, the aqueous phase W₂ also includes sodium chloride, and the sodium chloride has a mass fraction of 0 - 10%.

In step (2.1), a concentration of risperidone in the oil phase O is 50-300 mg/mL; preferably 100-200 mg/mL.

In step (2.1), a concentration of the degradable material B in the oil phase O is 100-300 mg/mL; preferably 100-200 mg/mL.

In step (2.2), a mass fraction of PVA in the aqueous phase W is 0.1-1.2%.

In some preferred embodiments, the aqueous phase W also includes sodium chloride, and the sodium chloride has a mass fraction of 0-10%.

In step (3), the organic solvent B is N-methyl pyrrolidone.

In step (3), a mass fraction of the degradable material B in the solution is 10-25%; preferably 10%-15%.

In step (3), the phosphate buffer has a pH of 7.4.

In step (4), an amount of the polymer blank particles accounts for 5-10% of the total.

In step (4), an amount of the lubricant accounts for 0-0.05% of the total.

In step (5), the irradiation sterilization is carried out under an intensity of 10-25 KGy.

The present application also provides the application of the naltrexone compound sustained-release implant in the preparation of drugs for treating or relieving amphetamine-type drug addiction.

Compared to the prior art, the present application has beneficial effects as follows:
(1) In the naltrexone compound sustained-release implant provided herein, a mass ratio of naltrexone to risperidone is (20-30):1, and within this ratio, the naltrexone compound sustained-release implant can effectively inhibit amphetamine type drug relapse while reducing side effects of risperidone;
(2) In the process of implementation of this application, by controlling a molecular weight of the degradable material A in the naltrexone microspheres to be 10W-15W, and a molecular weight of the degradable material B in the risperidone microspheres to be 2W-4W, within these ratios, the naltrexone compound sustained-release implant can synchronous release naltrexone and risperidone, since naltrexone has a water solubility of 3.07 mg/mL, a logP of 1.92; and risperidone has a water solubility of 0.171 mg/mL, a logP of 3.27. The inventor speeds up the release rate of risperidone by reducing a molecular weight of the polymer in risperidone microspheres during the implementation process, so as to synchronously release risperidone with naltrexone.
(3) It is widely known for those skilled in the art that high magnesium content has inhibitory effects on nerves and heart, and a mass ratio of naltrexone to risperidone disclosed in the prior art is (5-10):1, but the prepared microsphere is poor in compressibility, which requires adding a large amount of magnesium stearate lubrication to achieve continuous processing, thus there will be a large amount of magnesium ion Mg²⁺, which has a risk of exceeding the body's acceptable limit (0.5 mg/day). In the present application, by controlling a mass ratio of naltrexone to risperidone to be (20-30):1, and adjusting the types of excipients and preparation process, the active ingredients can be uniformly released. In addition, it also improves the compressibility of the preparation, reduces the amount of magnesium stearate and improves the release stability of magnesium stearate, thus improving the safety of the preparation in vivo.
(4) Since the solubility of naltrexone is higher than that of risperidone, a W/O/O/W process is adopted herein to prepare naltrexone microspheres, and an O/W process is adopted to prepare risperidone microspheres, to achieve membrane-controlled drug release of each particle in one step. In addition, PLA blank particles are further added in the process, which improves the compressibility of the preparation.
(5) Since when the polymer blank particles are pressed, coated polymer may be dispersed to various parts and surface layers of the cylindrical tablet, residual N-methyl pyrrolidone will be volatilized by irradiation, PLA in the tablet may form a network structure, and the surface layer forms a dense film, which can lock in the drug and reduce the possibility of burst release. The intelligent application of irradiation makes the film layer denser, which further achieves synchronous release of naltrexone and risperidone.

### Brief Description of the Drawings

Fig. 1 shows dissolution profile of a sustained release implant prepared in Example 1;
Fig. 2 shows dissolution profile of a sustained release implant prepared in Example 2;
Fig. 3 shows dissolution profile of a sustained release implant prepared in Example 3;
Fig. 4 shows dissolution profile of a sustained release implant prepared in Example 4;
Fig. 5 shows dissolution profile of a sustained release implant prepared in Example 5;
Fig. 6 shows dissolution profile of a sustained release implant prepared in Comparative Example 2;
Fig. 7 shows dissolution profile of a sustained release implant prepared in Comparative Example 3;
Fig. 8 shows dissolution profile of a sustained release implant prepared in Comparative Example 4;
Fig. 9 shows dissolution profile of a sustained release implant prepared in Comparative Example 5;
Fig. 10 shows magnesium ion residue curve of a sustained release implant prepared in Example 1;
Fig. 11 shows magnesium ion residue curve of a sustained release implant prepared in Comparative Example 1;
Fig. 12 is an electron micrograph of PLA blank particles prepared in Example 1;
Fig. 13 is an electron micrograph of naltrexone microspheres prepared in Example 1; and
Fig. 14 shows results of subject's conditioned place preference.

### Detailed Description of the Embodiments

The features mentioned the above, or in embodiments, may be arbitrarily combined. All the features explained herein may be used with any methodological forms, and each feature revealed herein may be replaced by any substitutive feature that provides the same, equal or similar purpose. Thus, unless otherwise specified, the features revealed are only general examples of equal or similar features.

The application is further elaborated in combination with specific examples. These examples are used only for illustrating the application and not to limit the scope thereof. If the experimental methods used in the examples are not specifically described, they are carried out usually in accordance with conventional conditions or in accordance with conditions suggested by the manufacturer. All percentages and fractions are by weight unless otherwise stated.

Unless otherwise defined, all professional and scientific terms used herein have the same meaning as those familiar to those skilled in the art. In addition, any method or material similar or equal to the recorded content may be applied to the method of the application. The preferred implementation methods and materials described herein are only for demonstration.

The reagents used in the following examples of this application are general reagents in this field and may be purchased commercially.

### Example 1: A naltrexone compound sustained-release implant and a preparation method thereof

1. Preparation of naltrexone microspheres:
   (1.1) 0.15 g sodium chloride was dissolved in water at a concentration of 15 mg/mL, to obtain an aqueous phase W₁;
   (1.2) 3.5 g naltrexone and 3.5 g PLA with a molecular weight of 10W were dissolved in dichloromethane, to obtain an oil phase O₁ with a concentration of naltrexone of 150 mg/mL and a concentration of PLA of 150 mg/mL;
   (1.3) the aqueous phase W₁ was added to the oil phase O₁, ultrasonic-assisted emulsified to obtain a primary emulsion W₁/O₁;
   (1.4) 3.5 g PLA with a molecular weight of 10W was dissolved in dichloromethane, to obtain an oil phase O₂ with a concentration of PLA of 300 mg/mL;
   (1.5) the primary emulsion W₁/O₁ was added to the oil phase O₂ with a ratio of 1:5, ultrasonic-assisted emulsified to obtain a multiple emulsion W₁/O₁/O₂;
   (1.6) an aqueous solution of polyvinyl alcohol (PVA) with a mass fraction of 0.5% was prepared, which contained 5% sodium chloride, to obtain an aqueous phase W₂;
   (1.7) the multiple emulsion W₁/O₁/O₂ was dispersed into W₂, stirred continuously at 2000 r/min, and then continuously stirred at 500 r/min for 24 hours until the organic solvent was volatilized, filtered, microspheres were collected and dried to obtain the naltrexone microspheres.
2. Preparation of risperidone microspheres:
   (2.1) 3 g risperidone and 3 g PLA with a molecular weight of 2W were dissolved in dichloromethane, to obtain an oil phase with a concentration of risperidone of 150 mg/mL and a concentration of PLA of 150 mg/mL;
   (2.2) an aqueous solution of PVA with a mass fraction of 0.5% was prepared, which contained 5% sodium chloride, to obtain an aqueous phase.;
   (2.3) the oil phase was dispersed into the aqueous phase, stirred at 1000 r/min, and then continuously stirred at 500 r/min at 35°C for 24 hours until the organic solvent was volatilized, filtered, microspheres were collected and dried to obtain the risperidone microspheres.
3. Preparation of PLA blank particles:
   1 g PLA with a molecular weight of 2W was dissolved in N-methyl pyrrolidone to obtain a PLA solution with a concentration of 15%; the PLA solution was dispersed in a phosphate buffer with a pH of 7.4, to obtain polymer blank particles. D50 particle size of the polymer blank particles was 200±30µm.
4. Blending and tableting
   The naltrexone microspheres and the risperidone microspheres were blended at a weight ratio of active substances of 20:1, then blended with 5% of the polymer blank particles and 0.03% of magnesium stearate, a resulting product was pressed by a tablet press to obtain cylindrical tablets with diameter of 7 mm, each containing 50 mg naltrexone and 2.5 mg risperidone.
5. Irradiation sterilization
   The pressed cylindrical tablets were sterilized by irradiation at 25 KGy, to obtain the naltrexone compound sustained-release implant.

### Example 2: A naltrexone compound sustained-release implant and a preparation method thereof

1. Preparation of naltrexone microspheres:
   (1.1) 0.2 g sodium bicarbonate was dissolved in water at a concentration of 20 mg/mL, to obtain an aqueous phase W₁;
   (1.2) 3.6 g naltrexone and 2.4 g PLA with a molecular weight of 15W were dissolved in dichloromethane, to obtain an oil phase O₁ with a concentration of naltrexone of 180 mg/mL and a concentration of PLA of 120 mg/mL;
   (1.3) the aqueous phase W₁ was added to the oil phase O₁, ultrasonic-assisted emulsified to obtain a primary emulsion W₁/O₁;
   (1.4) 2.4 g PLA with a molecular weight of 15W was dissolved in dichloromethane, to obtain an oil phase O₂ with a concentration of PLA of 300 mg/mL;
   (1.5) the primary emulsion W₁/O₁ was added to the oil phase O₂ with a ratio of 1:3, ultrasonic-assisted emulsified to obtain a multiple emulsion W₁/O₁/O₂;
   (1.6) an aqueous solution of PVA with a mass fraction of 0.3% was prepared, which contained 10% of sodium chloride, to obtain an aqueous phase W₂;
   (1.7) the multiple emulsion W₁/O₁/O₂ was dispersed into W₂, stirred continuously at 2000 r/min, and then continuously stirred at 500 r/min for 24 hours until the organic solvent was volatilized, filtered, microspheres were collected and dried to obtain the naltrexone microspheres.
2. Preparation of risperidone microspheres:
   (2.1) 1.5 g risperidone and 1.2 g PLA with a molecular weight of 4W were dissolved in dichloromethane, to obtain an oil phase with a concentration of risperidone of 150 mg/mL and a concentration of PLA of 120 mg/mL;
   (2.2) an aqueous solution of PVA with a mass fraction of 0.7% was prepared, which contained 3% of sodium chloride, to obtain an aqueous phase.;
   (2.3) the oil phase was dispersed into the aqueous phase, stirred at 1000 r/min, and then continuously stirred at 500 r/min at 35 °C for 24 hours until the organic solvent was volatilized, filtered, microspheres were collected and dried to obtain the risperidone microspheres.
3. Preparation of PLA blank particles:
   1 g PLA with a molecular weight of 4W was dissolved in N-methyl pyrrolidone to obtain a PLA solution with a concentration of 10%; the PLA solution was dispersed in a phosphate buffer with a pH of 7.4, to obtain polymer blank particles. D50 particle size of the polymer blank particles was 220±27 µm.
4. Blending and tableting
   The naltrexone microspheres and the risperidone microspheres were blended at a weight ratio of active substances of 30:1, then blended with 5% of the polymer blank particles and 0.04% of magnesium stearate, a resulting product was pressed by a tablet press to obtain cylindrical tablets with diameter of 7 mm, each containing 50 mg naltrexone and 1.67 mg risperidone.
5. Irradiation sterilization
   The pressed cylindrical tablets were sterilized by irradiation at 25 KGy, to obtain the naltrexone compound sustained-release implant.

### Example 3: A naltrexone compound sustained-release implant and a preparation method thereof

1. Preparation of naltrexone microspheres:
   (1.1) 0.15 g disodium hydrogencarbonate was dissolved in water at a concentration of 15 mg/mL, to obtain an aqueous phase W₁;
   (1.2) 3 g naltrexone and 3 g PLA with a molecular weight of 13W were dissolved in dichloromethane, to obtain an oil phase O₁ with a concentration of naltrexone of 150 mg/mL and a concentration of PLA of 150 mg/mL;
   (1.3) the aqueous phase W₁ was added to the oil phase O₁, ultrasonic-assisted emulsified to obtain a primary emulsion W₁/O₁;
   (1.4) 2.5 g PLA with a molecular weight of 13W was dissolved in dichloromethane, to obtain an oil phase O₂ with a concentration of PLA of 300 mg/mL;
   (1.5) the primary emulsion W₁/O₁ was added to the oil phase O₂ with a ratio of 1:4, ultrasonic-assisted emulsified to obtain a multiple emulsion W₁/O₁/O₂;
   (1.6) an aqueous solution of PVA with a mass fraction of 0.8% was prepared, which contained 0.2% of sodium chloride, to obtain an aqueous phase W₂;
   (1.7) the multiple emulsion W₁/O₁/O₂ was dispersed into W₂, stirred continuously at 2000 r/min, and then continuously stirred at 500 r/min for 24 hours until the organic solvent was volatilized, filtered, microspheres were collected and dried to obtain the naltrexone microspheres.
2. Preparation of risperidone microspheres:
   (2.1) 2.25 g risperidone and 2.25 g PLA with a molecular weight of 3W were dissolved in dichloromethane, to obtain an oil phase with a concentration of risperidone of 150 mg/mL and a concentration of PLA of 150 mg/mL;
   (2.2) an aqueous solution of PVA with a mass fraction of 0.9% was prepared, which contained 4% of sodium chloride, to obtain an aqueous phase.;
   (2.3) the oil phase was dispersed into the aqueous phase, stirred at 1000 r/min, and then continuously stirred at 500 r/min at 35 °C for 24 hours until the organic solvent was volatilized, filtered, microspheres were collected and dried to obtain the risperidone microspheres.
3. Preparation of PLA blank particles:
   0.8 g PLA with a molecular weight of 3W was dissolved in N-methyl pyrrolidone to obtain a PLA solution with a concentration of 10%; the PLA solution was dispersed in a phosphate buffer with a pH of 7.4, to obtain polymer blank particles. D50 particle size of the polymer blank particles was 204±26 µm.
4. Blending and tableting
   The naltrexone microspheres and the risperidone microspheres were blended at a weight ratio of active substances of 20:1, then blended with 6% of the polymer blank particles and 0.01% of magnesium stearate, a resulting product was pressed by a tablet press to obtain cylindrical tablets with diameter of 7 mm, each containing 50 mg naltrexone and 2.5 mg risperidone.
5. Irradiation sterilization
   The pressed cylindrical tablets were sterilized by irradiation at 20 KGy, to obtain the naltrexone compound sustained-release implant.

### Example 4: A naltrexone compound sustained-release implant and a preparation method thereof

1. Preparation of naltrexone microspheres:
   (1.1) 0.4 g potassium chloride was dissolved in water at a concentration of 20 mg/mL, to obtain an aqueous phase W₁;
   (1.2) 3 g naltrexone and 2.4 g PLA with a molecular weight of 11W were dissolved in dichloromethane, to obtain an oil phase O₁ with a concentration of naltrexone of 200 mg/mL and a concentration of PLA of 160 mg/mL;
   (1.3) the aqueous phase W₁ was added to the oil phase O₁, ultrasonic-assisted emulsified to obtain a primary emulsion W₁/O₁;
   (1.4) 2.7 g PLA with a molecular weight of 11W was dissolved in dichloromethane, to obtain an oil phase O₂ with a concentration of PLA of 300 mg/mL;
   (1.5) the primary emulsion W₁/O₁ was added to the oil phase O₂ with a ratio of 1:4, ultrasonic-assisted emulsified to obtain a multiple emulsion W₁/O₁/O₂;
   (1.6) an aqueous solution of PVA with a mass fraction of 1.2% was prepared, which contained 8% of sodium chloride, to obtain an aqueous phase W₂;
   (1.7) the multiple emulsion W₁/O₁/O₂ was dispersed into W₂, stirred continuously at 2000 r/min, and then continuously stirred at 500 r/min for 24 hours until the organic solvent was volatilized, filtered, microspheres were collected and dried to obtain the naltrexone microspheres.
2. Preparation of risperidone microspheres:
   (2.1) 1.8 g risperidone and 1.2 g PLA with a molecular weight of 4W were dissolved in dichloromethane, to obtain an oil phase with a concentration of risperidone of 180 mg/mL and a concentration of PLA of 120 mg/mL;
   (2.2) an aqueous solution of PVA with a mass fraction of 0.6% was prepared, which contained 6% of sodium chloride, to obtain an aqueous phase.;
   (2.3) the oil phase was dispersed into the aqueous phase, stirred at 1000 r/min, and then continuously stirred at 500 r/min at 35 °C for 24 hours until the organic solvent was volatilized, filtered, microspheres were collected and dried to obtain the risperidone microspheres.
3. Preparation of PLA blank particles:
   0.5 g PLA with a molecular weight of 4W was dissolved in N-methyl pyrrolidone to obtain a PLA solution with a concentration of 10%; the PLA solution was dispersed in a phosphate buffer with a pH of 7.4, to obtain polymer blank particles. D50 particle size of the polymer blank particles was 211±21µm.
4. Blending and tableting
   The naltrexone microspheres and the risperidone microspheres were blended at a weight ratio of active substances of 20:1, then blended with 6% of the polymer blank particles and 0.045% of magnesium stearate, a resulting product was pressed by a tablet press to obtain cylindrical tablets with diameter of 7 mm, each containing 50 mg naltrexone and 2.5 mg risperidone.
5. Irradiation sterilization
   The pressed cylindrical tablets were sterilized by irradiation at 15 KGy, to obtain the naltrexone compound sustained-release implant.

### Example 5: A naltrexone compound sustained-release implant and a preparation method thereof

1. Preparation of naltrexone microspheres:
   (1.1) 0.15 g sodium chloride was dissolved in water at a concentration of 15 mg/mL, to obtain an aqueous phase W₁;
   (1.2) 3.5 g naltrexone and 3.5 g PLA with a molecular weight of 11W were dissolved in dichloromethane, to obtain an oil phase O₁ with a concentration of naltrexone of 150 mg/mL and a concentration of PLA of 150 mg/mL;
   (1.3) the aqueous phase W₁ was added to the oil phase O₁, ultrasonic-assisted emulsified to obtain a primary emulsion W₁/O₁;
   (1.4) 3.5 g PLA with a molecular weight of 11W was dissolved in dichloromethane, to obtain an oil phase O₂ with a concentration of PLA of 300 mg/mL;
   (1.5) the primary emulsion W₁/O₁ was added to the oil phase O₂ with a ratio of 1:5, ultrasonic-assisted emulsified to obtain a multiple emulsion W₁/O₁/O₂;
   (1.6) an aqueous solution of PVA with a mass fraction of 0.5% was prepared, which contained 5% sodium chloride, to obtain an aqueous phase W₂;
   (1.7) the multiple emulsion W₁/O₁/O₂ was dispersed into W₂, stirred continuously at 2000 r/min, and then continuously stirred at 500 r/min for 24 hours until the organic solvent was volatilized, filtered, microspheres were collected and dried to obtain the naltrexone microspheres.
2. Preparation of risperidone microspheres:
   (2.1) 3 g risperidone and 3 g PLA with a molecular weight of 2W were dissolved in dichloromethane, to obtain an oil phase with a concentration of risperidone of 150 mg/mL and a concentration of PLA of 150 mg/mL;
   (2.2) an aqueous solution of PVA with a mass fraction of 0.5% was prepared, which contained 5% sodium chloride, to obtain an aqueous phase.;
   (2.3) the oil phase was dispersed into the aqueous phase, stirred at 1000 r/min, and then continuously stirred at 500 r/min at 35 °C for 24 hours until the organic solvent was volatilized, filtered, microspheres were collected and dried to obtain the risperidone microspheres.
3. Preparation of PLA blank particles:
   1g PLA with a molecular weight of 2W was dissolved in N-methyl pyrrolidone to obtain a PLA solution with a concentration of 15%; the PLA solution was dispersed in a phosphate buffer with a pH of 7.4, to obtain polymer blank particles. D50 particle size of the polymer blank particles was 203±15µm.
4. Blending and tableting
   The naltrexone microspheres and the risperidone microspheres were blended at a weight ratio of active substances of 30:1, then blended with 5% of the polymer blank particles and 0.015% of magnesium stearate, a resulting product was pressed by a tablet press to obtain cylindrical tablets with diameter of 7 mm, each containing 50 mg naltrexone and 1.67 mg risperidone.
5. Irradiation sterilization
   The pressed cylindrical tablets were sterilized by irradiation at 10 KGy, to obtain the naltrexone compound sustained-release implant.

### Comparative Example 1

1. Preparation of risperidone microspheres:
   (1.1) 0.6 g sodium chloride was dissolved in 4 mL pure water, to obtain an inner aqueous phase;
   (1.2) 3.5 g risperidone and PLA with a molecular weight of 8000 were dissolved in dichloromethane, to obtain an oil phase with a concentration of risperidone of 150 mg/mL and a concentration of PLA of 150 mg/mL;
   (1.3) the inner aqueous phase was added to the oil phase, ultrasonic-assisted emulsified to obtain a primary emulsion;
   (1.4) the primary emulsion was added dropwise rapidly to an aqueous solution of PVA with a mass fraction of 0.5% containing 5% sodium chloride, stirred evenly at 1000 r/min, and then continuously stirred at 200 r/min at 35 °C for 24 hours until the organic solvent was volatilized, microspheres were collected and dried to obtain the naltrexone microspheres
2. Preparation of naltrexone microspheres:
   (2.1) 3.5 g naltrexone and 3.5 g PLA with a molecular weight of 8000 were dissolved in dichloromethane, to obtain an oil phase with a concentration of naltrexone of 150 mg/mL and a concentration of PLA of 150 mg/mL;
   (2.2) an aqueous solution of PVA with a mass fraction of 0.5% was prepared, which contained 10% sucrose, to obtain an aqueous phase.;
   (2.3) the oil phase was dispersed into the aqueous phase, stirred evenly at 1000 r/min, and then continuously stirred at 200 r/min at 35 °C for 2 hours until the organic solvent was volatilized, microspheres were collected, washed and dried to obtain the risperidone microspheres.
3. Blending and tableting
   The naltrexone microspheres and the risperidone microspheres were blended at a weight ratio of active substances of 10:1, then blended with 0.05% of magnesium stearate, a resulting product was pressed to obtain tablets, each containing 50 mg naltrexone and 5 mg risperidone.
4. Spray coating

### Comparative Example 2

1. Preparation of naltrexone microspheres:
   (1.1) 0.15g sodium chloride was dissolved in water at a concentration of 15 mg/mL, to obtain an aqueous phase W₁;
   (1.2) 3.5 g naltrexone and 3.5 g PLA with a molecular weight of 10W were dissolved in dichloromethane, to obtain an oil phase O₁ with a concentration of naltrexone of 150 mg/mL and a concentration of PLA of 150 mg/mL;
   (1.3) the aqueous phase W₁ was added to the oil phase O₁, ultrasonic-assisted emulsified to obtain a primary emulsion;
   (1.4) 3.5 g PLA with a molecular weight of 10W was dissolved in dichloromethane, to obtain an oil phase O₂ with a concentration of PLA of 300 mg/mL;
   (1.5) the primary emulsion was added to the oil phase O₂ at a ratio of 1:5, ultrasonic-assisted emulsified to obtain a multiple emulsion W₁/O₁/O₂;
   (1.6) an aqueous solution of PVA with a mass fraction of 0.5% was prepared, which contained 5% sodium chloride, to obtain an aqueous phase W₂;
   (1.7) the multiple emulsion W₁/O₁/O₂ was dispersed into W₂, stirred continuously at 2000 r/min, and then continuously stirred at 500 r/min for 24 hours until the organic solvent was volatilized, filtered, microspheres were collected and dried to obtain the naltrexone microspheres.
2. Preparation of risperidone microspheres:
   (2.1) 3 g risperidone and 3 g PLA with a molecular weight of 2W were dissolved in dichloromethane, to obtain an oil phase with a concentration of risperidone of 150 mg/mL and a concentration of PLA of 150 mg/mL;
   (2.2) an aqueous solution of PVA with a mass fraction of 0.5% was prepared, which contained 5% sodium chloride, to obtain an aqueous phase.;
   (2.3) the oil phase was dispersed into the aqueous phase, stirred at 1000 r/min, and then continuously stirred at 500 r/min at 35 °C for 24 hours until the organic solvent was volatilized, filtered, microspheres were collected and dried to obtain the risperidone microspheres.
3. Preparation of PLA blank particles:
   1 g PLA with a molecular weight of 2W was dissolved in N-methyl pyrrolidone to obtain a PLA solution with a concentration of 15%; the PLA solution was dispersed in a phosphate buffer with a pH of 7.4, to obtain polymer blank particles. D50 particle size of the polymer blank particles was 200±30µm.
4. Blending and tableting
   The naltrexone microspheres and the risperidone microspheres were blended at a weight ratio of active substances of 40:1, then blended with 5% of the polymer blank particles and 0.1% of magnesium stearate, a resulting product was pressed by a tablet press to obtain cylindrical tablets with diameter of 7 mm, each containing 50 mg naltrexone and 1.25 mg risperidone.
5. Irradiation sterilization
   The pressed cylindrical tablets were sterilized by irradiation at 25 KGy, to obtain the naltrexone compound sustained-release implant.

### Comparative Example 3

1. Preparation of naltrexone microspheres:
   (1.1) 3.5 g naltrexone and 3.5 g PLA with a molecular weight of 10W were dissolved in dichloromethane, to obtain an oil phase with a concentration of naltrexone of 150 mg/mL and a concentration of PLA of 150 mg/mL;
   (1.2) an aqueous solution of PVA with a mass fraction of 0.5% was prepared, which contained 10% of sucrose, to obtain an aqueous phase.;
   (1.3) the oil phase was added dropwise rapidly to the aqueous solution, stirred evenly at 1000 r/min, and then continuously stirred at 200 r/min at 35 °C for 2 hours until the organic solvent was volatilized, microspheres were collected, washed and dried to obtain the naltrexone microspheres.
2. Preparation of risperidone microspheres:
   (2.1) 0.6 g sodium chloride was dissolved in 4 mL pure water, to obtain an inner aqueous phase;
   (2.2) 3 g risperidone and 3 g PLA with a molecular weight of 2W were dissolved in dichloromethane, to obtain an oil phase with a concentration of risperidone of 150 mg/mL and a concentration of PLA of 150 mg/mL;
   (2.3) the inner aqueous phase was added to the oil phase, ultrasonic-assisted emulsified to obtain a primary emulsion;
   (2.4) an aqueous solution of PVA with a mass fraction of 0.5% was prepared, which contained 5% sodium chloride, to obtain an aqueous phase.;
   (2.5) the primary emulsion was added dropwise rapidly to the aqueous solution, stirred evenly at 1000 r/min, and then continuously stirred at 200 r/min at 35 °C for 24 hours until the organic solvent was volatilized, microspheres were collected, washed and dried to obtain the risperidone microspheres.
3. Preparation of PLA blank particles:
   PLA with a molecular weight of 2W was dissolved in N-methyl pyrrolidone to obtain a PLA solution with a concentration of 15%; the PLA solution was dispersed in a phosphate buffer with a pH of 7.4, to obtain polymer blank particles. D50 particle size of the polymer blank particles was 200±30µm.
4. Blending and tableting
   The naltrexone microspheres and the risperidone microspheres were blended at a weight ratio of active substances of 20:1, then blended with 5% of the polymer blank particles and 0.1% of magnesium stearate, a resulting product was pressed by a tablet press to obtain cylindrical tablets with diameter of 7 mm, each containing 50 mg naltrexone and 2.5 mg risperidone.
5. Irradiation sterilization
   The pressed cylindrical tablets were sterilized by irradiation at 25 KGy, to obtain the naltrexone compound sustained-release implant.

### Comparative Example 4

1. Preparation of naltrexone microspheres:
   (1.1) 0.15 g sodium chloride was dissolved in water at a concentration of 15 mg/mL, to obtain an aqueous phase W₁;
   (1.2) 3.5g naltrexone and 3.5g PLA with a molecular weight of 10W were dissolved in dichloromethane, to obtain an oil phase O₁ with a concentration of naltrexone of 150 mg/mL and a concentration of PLA of 150 mg/mL;
   (1.3) the aqueous phase W₁ was added to the oil phase O₁, ultrasonic-assisted emulsified to obtain a primary emulsion W₁/O₁;
   (1.4) 3.5 g PLA with a molecular weight of 10W was dissolved in dichloromethane, to obtain an oil phase O₂ with a concentration of PLA of 300 mg/mL;
   (1.5) the primary emulsion W₁/O₁ was added to the oil phase O₂ with a ratio of 1:5, ultrasonic-assisted emulsified to obtain a multiple emulsion W₁/O₁/O₂;
   (1.6) an aqueous solution of PVA with a mass fraction of 0.5% was prepared, which contained 5% sodium chloride, to obtain an aqueous phase W₂;
   (1.7) the multiple emulsion W₁/O₁/O₂ was dispersed into W₂, stirred evenly at 2000 r/min, and then continuously stirred at 500 r/min for 24 hours until the organic solvent was volatilized, microspheres were collected, washed and dried to obtain the naltrexone microspheres.
2. Preparation of risperidone microspheres:
   (2.1) 3 g risperidone and 3 g PLA with a molecular weight of 2W were dissolved in dichloromethane, to obtain an oil phase with a concentration of risperidone of 150 mg/mL and a concentration of PLA of 150 mg/mL;
   (2.2) an aqueous solution of PVA with a mass fraction of 0.5% was prepared, which contained 5% sodium chloride, to obtain an aqueous phase.;
   (2.3) the oil phase was dispersed into the aqueous phase, stirred evenly at 1000 r/min, and then continuously stirred at 500 r/min at 35 °C for 24 hours until the organic solvent was volatilized, microspheres were collected, washed and dried to obtain the risperidone microspheres.
3. Preparation of PLA blank particles:
   1 g PLA with a molecular weight of 2W was dissolved in N-methyl pyrrolidone to obtain a PLA solution with a concentration of 15%; the PLA solution was dispersed in a phosphate buffer with a pH of 7.4, to obtain polymer blank particles. D50 particle size of the polymer blank particles was 200±30µm.
4. Blending and tableting
   The naltrexone microspheres and the risperidone microspheres were blended at a weight ratio of active substances of 10:1, then blended with 5% of the polymer blank particles and 0.03% of magnesium stearate, a resulting product was pressed by a tablet press to obtain cylindrical tablets with diameter of 7 mm, each containing 50 mg naltrexone and 5 mg risperidone.
5. Irradiation sterilization
   The pressed cylindrical tablets were sterilized by irradiation at 25 KGy, to obtain the naltrexone compound sustained-release implant.

### Comparative Example 5

1. Preparation of naltrexone microspheres:
   (1.1) 0.15 g sodium chloride was dissolved in water at a concentration of 15 mg/mL, to obtain an aqueous phase W₁;
   (1.2) 3.5 g naltrexone and 3.5 g PLA with a molecular weight of 4W were dissolved in dichloromethane, to obtain an oil phase O₁ with a concentration of naltrexone of 150 mg/mL and a concentration of PLA of 150 mg/mL;
   (1.3) the aqueous phase W₁ was added to the oil phase O₁, ultrasonic-assisted emulsified to obtain a primary emulsion W₁/O₁;
   (1.4) 3.5 g PLA with a molecular weight of 4W was dissolved in dichloromethane, to obtain an oil phase O₂ with a concentration of PLA of 300 mg/mL;
   (1.5) the primary emulsion W₁/O₁ was added to the oil phase O₂ with a ratio of 1:5, ultrasonic-assisted emulsified to obtain a multiple emulsion W₁/O₁/O₂;
   (1.6) an aqueous solution of PVA with a mass fraction of 0.5% was prepared, which contained 5% sodium chloride, to obtain an aqueous phase W₂;
   (1.7) the multiple emulsion W₁/O₁/O₂ was dispersed into W₂, stirred evenly at 2000 r/min, and then continuously stirred at 500 r/min for 24 hours until the organic solvent was volatilized, microspheres were collected, washed and dried to obtain the naltrexone microspheres.
2. Preparation of risperidone microspheres:
   (2.1) 3 g risperidone and 3 g PLA with a molecular weight of 4W were dissolved in dichloromethane, to obtain an oil phase with a concentration of risperidone of 150 mg/mL and a concentration of PLA of 150 mg/mL;
   (2.2) an aqueous solution of PVA with a mass fraction of 0.5% was prepared, which contained 5% sodium chloride, to obtain an aqueous phase.;
   (2.3) the oil phase was dispersed into the aqueous phase, stirred evenly at 1000 r/min, and then continuously stirred at 500 r/min at 35 °C for 24 hours until the organic solvent was volatilized, microspheres were collected, washed and dried to obtain the risperidone microspheres.
3. Preparation of PLA blank particles:
   1 g PLA with a molecular weight of 4W was dissolved in N-methyl pyrrolidone to obtain a PLA solution with a concentration of 15%; the PLA solution was dispersed in a phosphate buffer with a pH of 7.4, to obtain polymer blank particles. D50 particle size of the polymer blank particles was 200±30µm.
4. Blending and tableting
   The naltrexone microspheres and the risperidone microspheres were blended at a weight ratio of active substances of 20:1, then blended with 5% of the polymer blank particles and 0.03% of magnesium stearate, a resulting product was pressed by a tablet press to obtain cylindrical tablets with diameter of 7 mm, each containing 50 mg naltrexone and 2.5 mg risperidone.
5. Irradiation sterilization
   The pressed cylindrical tablets were sterilized by irradiation at 25 KGy, to obtain the naltrexone compound sustained-release implant.

### Comparative Example 6: A preparation method of a naltrexone implant

1. Preparation of naltrexone microspheres:
   (1.1) 0.15 g sodium chloride was dissolved in water at a concentration of 15 mg/mL, to obtain an aqueous phase W₁;
   (1.2) 3.5 g naltrexone and 3.5 g PLA with a molecular weight of 10W were dissolved in dichloromethane, to obtain an oil phase O₁ with a concentration of naltrexone of 150 mg/mL and a concentration of PLA of 150 mg/mL;
   (1.3) the aqueous phase W₁ was added to the oil phase O₁, ultrasonic-assisted emulsified to obtain a primary emulsion W₁/O₁;
   (1.4) 3.5 g PLA with a molecular weight of 10W was dissolved in dichloromethane, to obtain an oil phase O₂ with a concentration of PLA of 300 mg/mL;
   (1.5) the primary emulsion W₁/O₁ was added to the oil phase O₂ with a ratio of 1:5, ultrasonic-assisted emulsified to obtain a multiple emulsion W₁/O₁/O₂;
   (1.6) an aqueous solution of PVA with a mass fraction of 0.5% was prepared, which contained 5% sodium chloride, to obtain an aqueous phase W₂;
   (1.7) the multiple emulsion W₁/O₁/O₂ was dispersed into W₂, stirred evenly at 2000 r/min, and then continuously stirred at 500 r/min for 24 hours until the organic solvent was volatilized, microspheres were collected, washed and dried to obtain the naltrexone microspheres.
2. Preparation of PLA blank particles:
   1 g PLA with a molecular weight of 2W was dissolved in N-methyl pyrrolidone to obtain a PLA solution with a concentration of 15%;
   the PLA solution was dispersed in a phosphate buffer with a pH of 7.4, to obtain polymer blank particles. D50 particle size of the polymer blank particles was 200±30µm.
3. Blending and tableting
   The naltrexone microspheres, 5% of the polymer blank particles and 0.03% of magnesium stearate were blended, a resulting product was pressed by a tablet press to obtain cylindrical tablets with diameter of 7 mm, each containing 50 mg naltrexone.
4. Irradiation sterilization
   The pressed cylindrical tablets were sterilized by irradiation at 25 KGy, to obtain the naltrexone implant.

### Comparative Example 7: A preparation method of a risperidone implant

1. Preparation of risperidone microspheres:
   (1.1) 3 g risperidone and 3 g PLA with a molecular weight of 2W were dissolved in dichloromethane, to obtain an oil phase with a concentration of risperidone of 150 mg/mL and a concentration of PLA of 150 mg/mL;
   (1.2) an aqueous solution of PVA with a mass fraction of 0.5% was prepared, which contained 5% sodium chloride, to obtain an aqueous phase.;
   (1.3) the oil phase was added rapidly into the aqueous phase, stirred evenly at 1000 r/min, and then continuously stirred at 500 r/min at 35 °C for 24 hours until the organic solvent was volatilized, microspheres were collected, washed and dried to obtain the risperidone microspheres.
2. Preparation of PLA blank particles:
   1 g PLA with a molecular weight of 2W was dissolved in N-methyl pyrrolidone to obtain a PLA solution with a concentration of 15%; the PLA solution was dispersed in a phosphate buffer with a pH of 7.4, to obtain polymer blank particles. D50 particle size of the polymer blank particles was 200±30µm.
3. Blending and tableting
   The risperidone microspheres, 5% of the polymer blank particles and 0.03% of magnesium stearate were blended, a resulting product was pressed by a tablet press to obtain cylindrical tablets with diameter of 7 mm, each containing 2.5 mg risperidone.
4. Irradiation sterilization
   The pressed cylindrical tablets were sterilized by irradiation at 25 KGy, to obtain the risperidone implant.

### Experimental Example 1: In vitro release curve

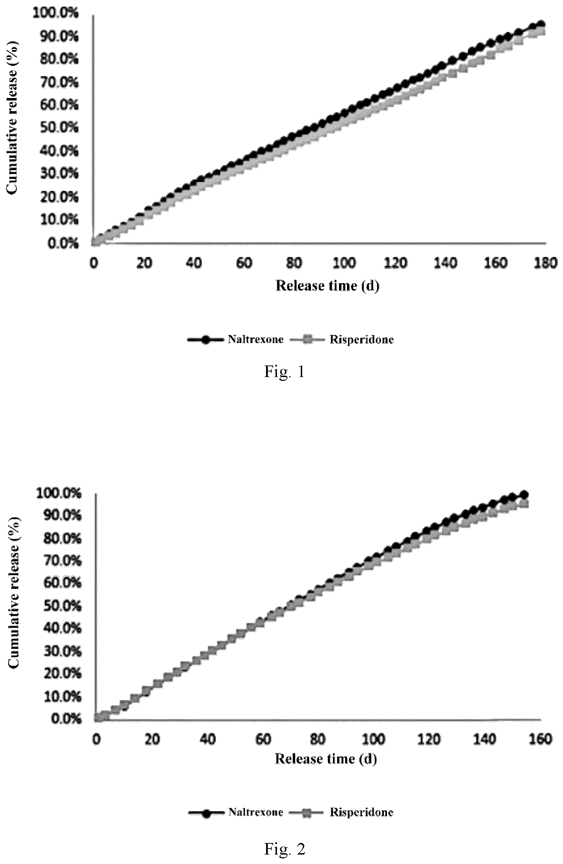

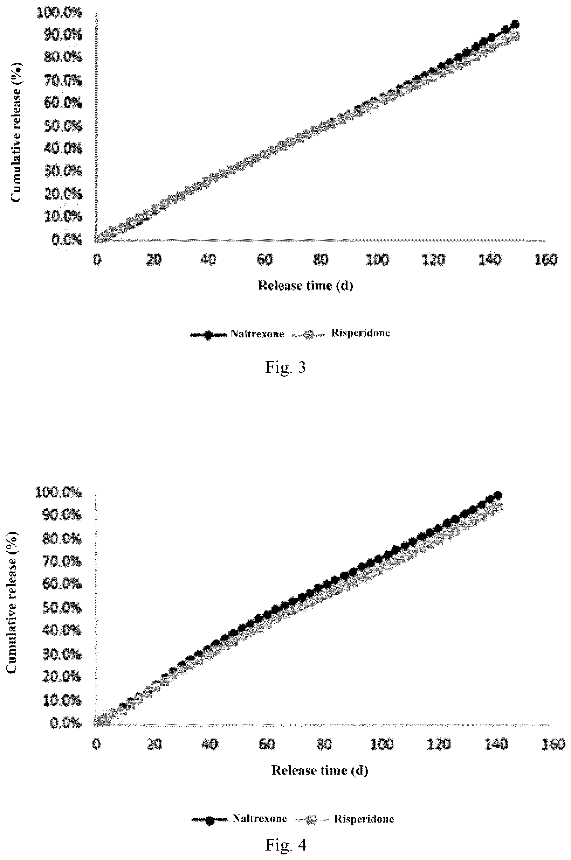

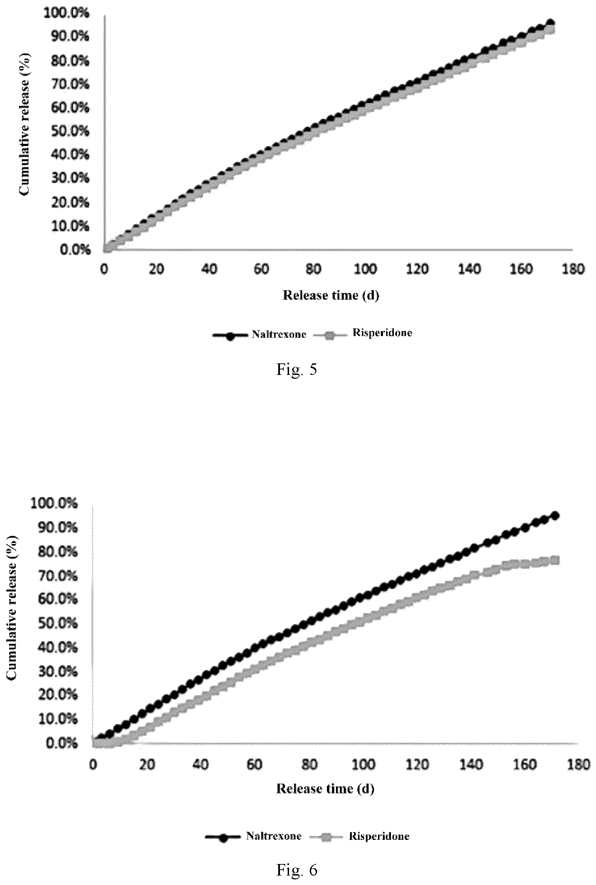

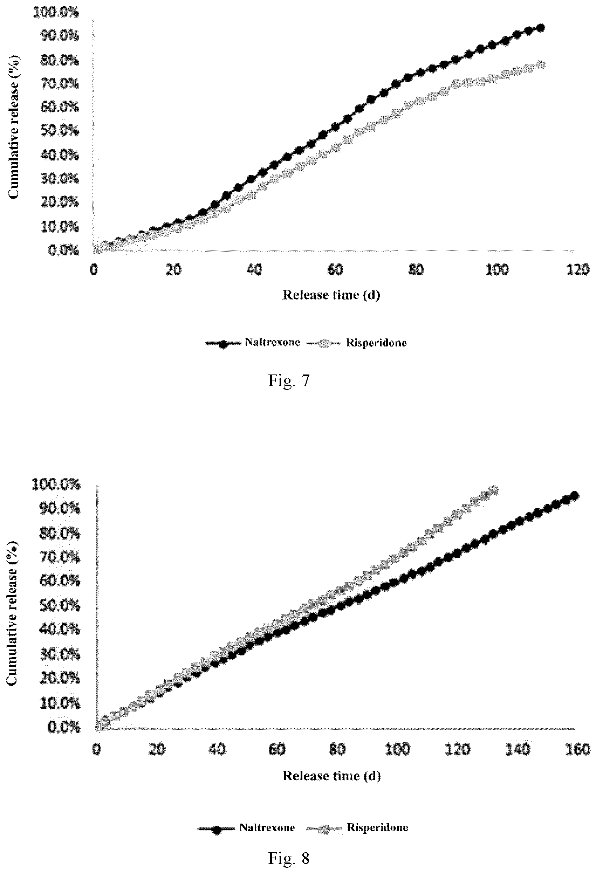

(1) Test sample: samples prepared in Examples 1-5 and Comparative Examples 2-5.
(2) Test method: the test samples were taken and placed in six 100 mL conical flask with stoppers, respectively, with 80 mL of 0.1 M potassium phosphate dibasic anhydrous solution (25 mL of 1 M sodium hydroxide solution was accurately weighed and placed in a 1000 mL volumetric flask, diluted with 0.1M potassium phosphate dibasic anhydrous solution, and adjusted the pH value to 11.2 ± 0.05) as solvents, sealed with the plugs, and placed in a water bath shaker at 37°C± 0.5°C, with an shaking frequency of 100 times per minute. On days 1, 3, 6, 9, 12, 15... 120, the medium was replaced, the solution was filtered, and subsequent filtrate was taken as the test solution, and the release medium was replenished in time in the operating container. In addition, an appropriate amount of naltrexone reference substance was taken, weighed accurately, dissolved in the release medium, and quantitatively diluted to make a solution containing about 50 µg naltrexone per 1 mL as the reference substance solution. The detection was carried out at a 280 nm wavelength according to high performance liquid chromatography (Chinese Pharmacopoeia 2020 (Edition IV) 0512 high performance liquid chromatography).
(3) The experimental results were shown in Figs. 1-9. Compared with Example 1, weight ratios of active substances in the naltrexone microspheres and risperidone microspheres in Comparative Examples 2 and 4 exceeded the scope of the application, resulting in a failure of synchronous release of naltrexone and risperidone. The preparation method in Comparative Example 3 was obviously different from that of the present application, and a stable release of naltrexone cannot be realized, nor can the synchronous release of naltrexone and risperidone be realized. PLA with the same molecular weight was used in Comparative Example 5, thus a release rate of naltrexone was obviously faster than that of peridone, and synchronous release cannot be achieved.

### Experimental Example 2: In vitro release curve of magnesium stearate (magnesium ion)

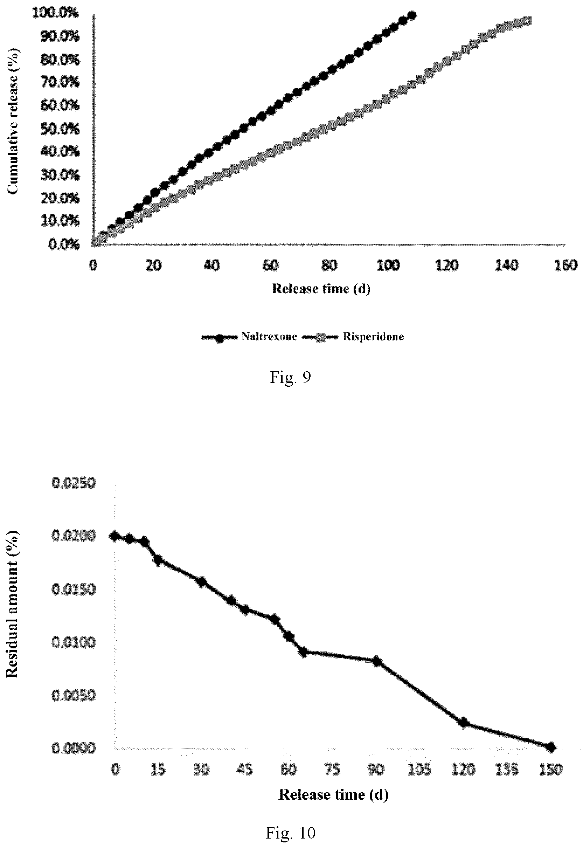

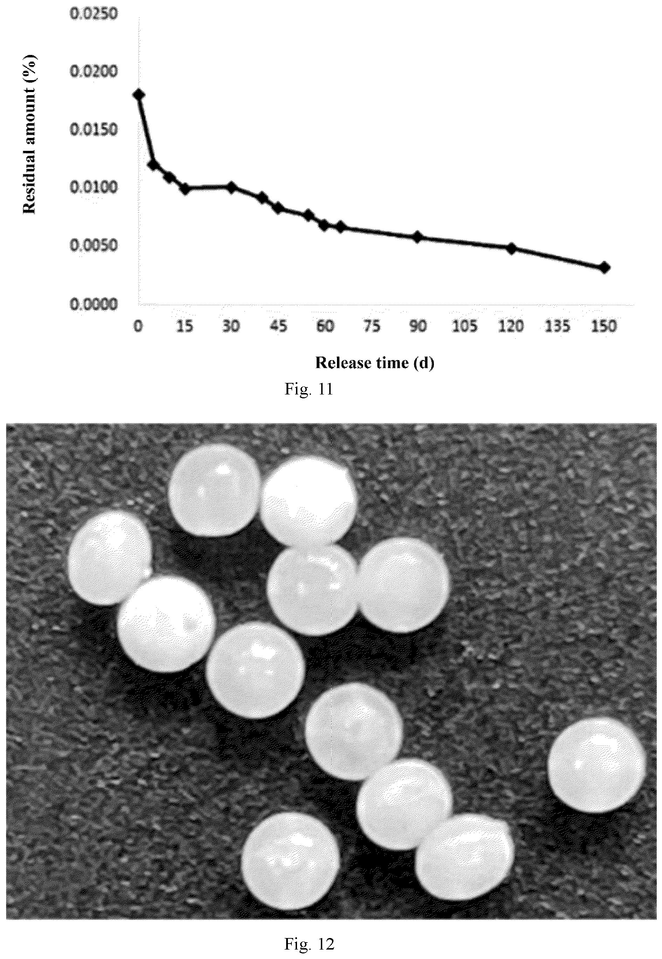

(1) Test sample: samples prepared in Example 1 and Comparative Example 1.
(2) Test method:
   A. Preparation of magnesium standard solution: 1 mL of magnesium single element standard solution (1000 µg/ml) was accurately measured, placed in a 100 mL volumetric flask, added with 2% nitric acid to obtain a solution containing 10 g magnesium per 1 mL, and shaken well. The standard solutions containing 0 µg, 0.4 µg, 0.8 µg, 1.0 µg and 1.5 µg per 1 mL were prepared by diluting the solution with 2% nitric acid.
   B. Preparation of test solution: two samples at each sampling time above were taken, freeze dried, and ground. About 250 mg of sample was weighed accurately, placed in a polytetrafluoroethylene digestion tank, added with 7 mL nitric acid, covered with an inner cover, tightened with an outer cover, placed in a microwave digestion system, and heated at 120°C for 15 min, 150°C for 15 min. After the digestion was complete, an inner tank was taken out and placed on an electric heating plate and slowly heated until a red-brown gas was dissipated. The digestion solution in the tank was transferred to a 50 mL volumetric flask and diluted to the scale with ultra-pure water, then shaken well, to obtain a test solution with a theoretical magnesium stearate concentration of 1 ug/mL.
   C. The standard solution of each concentration was determined by a coupled plasma atomic emission spectrometry, and a linear equation was obtained to calculate the residual element content of magnesium ion in a naltrexone implant.
(3) The experimental results were shown in Fig. 10 and Fig. 11. Compared with Comparative Example 1, in Example 1, the prescription and preparation process were adjusted, which improved the compressibility of the preparation, reduced the amount of magnesium stearate, made the release of magnesium stearate more stable, reduced the risk of excessive release of magnesium ion, and improved the safety of the implant in vivo.

### Experimental Example 3: Effect of compound implant of naltrexone on spontaneous activity

### (1) Test materials and groups:

Drugs and reagents: diazepam, and samples prepared in Example 1, Example 2, Comparative Example 2, Comparative Example 4, and Comparative Example 5.

Test animals: SPF-grade Sprague-Dawley (SD) rats, weighing 200 ± 20 g, and they were fed adaptively.

Animal grouping and administration: Animals were divided into 8 groups with 12 animals in each group, half male and half female. In the experiment, animals were divided into positive control group (diazepam group, with a dose of 2 mg/kg), negative control group (normal saline), Example 1 group, Example 2 group, Comparative Example 2 group, Comparative Example 4 group, Comparative Example 5 group (dose: 3 tablets/animal), and fasten for 12 h before administration. The dosage volume for rats was 10 mL/kg.

### (2) Test method:

On the day before the test sample was planted, the back of animals was shaved on both sides of the spine, exposing the skin to facilitate the operation of planting the test sample. After the animal was anesthetized with isoflurane, the local skin was disinfected with alcohol cotton ball, a wound about 0.5 cm in size was cut with a scalpel, a puncture needle was pushed forward about 3-4 cm, with left and right movement to ensure entering into a correct cortex, then tablets with corresponding dose was injected. The injection site was as far away from the spine as possible to avoid pain and injury to the animal due to movement and tablet friction. After sutures, anti-inflammatory powder was applied to the wound to ensure healing. After that, the wound was disinfected with iodophor daily until the wound was completely healed. Surgical instruments should be sterilized in advance.

The experiment was divided into three periods: adaptation period, pre-testing period and post-administration testing period.

Environmental adaptation: animals were kept for 3 days after arrival, 4 animals/cage, drank and ate freely, with a temperature of 20-24°C, a humidity of 40-60%, and a 12h light (L)/12h dark (D) rhythm cycle.

Open field adaptation: After environmental adaptation, single rat/mouse was put into a spontaneous activity box for 60 min to adapt to the experiment box, and the spontaneous activity within 60 min was recorded, the general adaptation was 2-3 days to reduce the impact of environmental stimulation.

Baseline test: Single rat/mouse was put into a spontaneous activity box for 40 min to adapt to the experimental box, and spontaneous activity within 40 min was recorded, then divided equally to groups according to basic spontaneous activity. Rats/mice with activities that are too high or too low (beyond Mean±2SD) were eliminated.

Drug administration test: On the second day after the completion of baseline spontaneous activity test, experimental animals were randomly divided into 8 groups according to the results of basic spontaneous activity test, with 12 animals in each group, then administrated with drugs for spontaneous activity test. Animals were single intragastric (ig) administrated. After 30 minutes of administration, single rat/mouse was put into a spontaneous activity box, spontaneous activity within 60 min in total was recorded, and the data were extracted every 10 minutes for analysis.

### (3) Data analysis

Data were expressed as Mean±SEM. Statistical analysis of time-segmented locomotor distance data: two-way repeated measures ANOVA and Bonferroni test were performed. Statistical analysis of the total motion distance data: t test was performed to compare the positive control group with the solvent control group; one-way ANOVA and Dunnett's t test were performed to compare different dose groups of the test sample with the solvent control group. P< 0.05 was taken as statistically significant difference, GraphPad Prism software was used for mapping and data analysis.

### (4) Experimental results were shown in Table 1

**Table 1: Effects of samples on total distance of activity in SD rats (mm: X ± SEM))**

| Group | Test |
|---|---|
| Solvent control group | 28431.73±9649.80 |
| Positive control group | 9698.94±902.16*** |
| Example 1 (20:1) | 15278.87±3062.54 |
| Example 2 (30:1) | 16117.01±1482.93 |
| Comparative Example 2 (40:1) | 16881.26±3010.00 |
| Comparative Example 4 (10:1) | 10801.26±2344.56** |
| Comparative Example 5 (20:1) | 14801.16±2843.51 |

| | |
|---|---|
| Compared with the solvent control group, *P≤0.05, **P≤ 0.01, ***P≤0.001 | |

As can be seen from the data in Table 1, in spontaneous activity test, results of Comparative Example 4 group and positive control group showed significant differences compared with the solvent control group, indicating that they had an effect on the central nervous system, which may be caused by the excessive content of risperidone in Comparative Example 4. There was no significant difference among other groups compared with the solvent control group, which could be used for further drug efficacy study.

Experimental Example 4: The compound implant of naltrexone blocks conditioned place preference caused by methamphetamine
(1) Test materials and groups: methamphetamine, samples prepared in Example 1, Example 2, Comparative Example 2, Comparative Example 5, Comparative Example 6 and Comparative Example 7.
   Experimental animals: SPF grade SD male rats, weighing 220-250 g, were randomly divided into groups, with 8 animals in each group.
   Experimental instrument: conditioned position preference instrument: The experiment was automatically controlled by computer. The device was a conditioned position preference box consisting of three boxes: two side boxes and a middle box. The three boxes were separated by movable partitions and were black inside and outside. The box A and box B were located on sides of the middle box and had the same size. The box A had nine square diodes that can emit yellow light on the side wall and a bottom made of stainless steel bar; box B had a bottom made of stainless steel grid. The time of rats in each box and the number of accesses may be transmitted to the computer through the data, and the behavioral data may be automatically collected and recorded.
(2) Test method:
   In this experiment, a morphine place preference model was established first, and the regression of the place preference model was observed after 10 days. Then, animals were subcutaneously implanted with the compound implant of naltrexone, and CPP reconstruction was performed with methamphetamine on 22 and 48 days after implanting the compound implant of naltrexone. The effect of CPP reconstruction in each group was observed.

### A. Establishment of CPP model

Basic value test: On the first day, channels among the three boxes were opened and CPP program was started on the computer. The rats were placed in the middle box and allowed to move freely in the three boxes for 15 minutes, and the time they stayed in each box was recorded synchronously by the computer. Conditioned place preference training: on days 2, 4, 6 and 8, rats in experimental groups were intraperitoneally injected with 0.1 mg/kg METH and put into the drug-paired chamber for 60 min; rats in the control groups were given water and put into the non-drug-paired chamber for 60 min. On days 3, 5, 7 and 9, rats in the experimental groups and control groups were given clean water, rats in experimental groups were put into the non-drug-paired chamber, and rats in control groups were put into the drug-paired chamber, for 60 min. The drug-paired chamber for each rat was fixed. Rats in each group were then returned to the cage.

CPP test: The CPP test was performed on day 10, similar to the basic value test phase. The channels among the three boxes were opened without any treatment, CPP program was started on the computer, the rats were put into the middle box and allowed to move freely in the three boxes for 15 minutes, and the time they stayed in each box was recorded synchronously by the computer.

The CPP score was defined as the difference between the time spent in the drug-paired chamber and the time spent in the non-drug-paired chamber. The measured value of post-CPP of rat in the drug-paired chamber was compared with the pre-CPP value to determine whether the rat formed CPP. The rats that did not form CPP were excluded according to the measured value of post-CPP, and the animals were matched into groups.

### B. CPP regression

On days 10-13 and 15-18 of the experiment, animals were injected intraperitoneally with normal saline, and on days 14 and 19, CPP tests were performed to test the rats' preference for the drug-paired chamber for 15 min, and CPP values were recorded. After the measurement for CPP regression was finished, animals in experimental groups were subcutaneously implanted with the corresponding test samples, which was regarded as day D0 of the test.

### C. CPP ignition

First ignition: On day D1, a small amount of METH was used for ignition and rats were put into the middle box to begin a 15-minute CPP value test.

Second ignition: Rats were injected with METH abdomen alternately from day D28 (rats in normal saline group were given normal saline), CPP training operation was performed, and CPP measurement was performed on day D21 (the method was the same as that of CPP model establishment).

Third ignition: Rats were injected with METH abdomen alternately from day D100 (rats in normal saline group were given normal saline), CPP training operation was performed, and CPP measurement was performed on day D42 (the method was the same as the above); no treatment was done to the rats at other time.

Indicator detection: After rats were trained, METH addiction was detected by a conditioned place preference box, and CPP Score reflected the formation of addictive behavior of rats. The increase of CPP Score indicated the formation of addictive behavior.

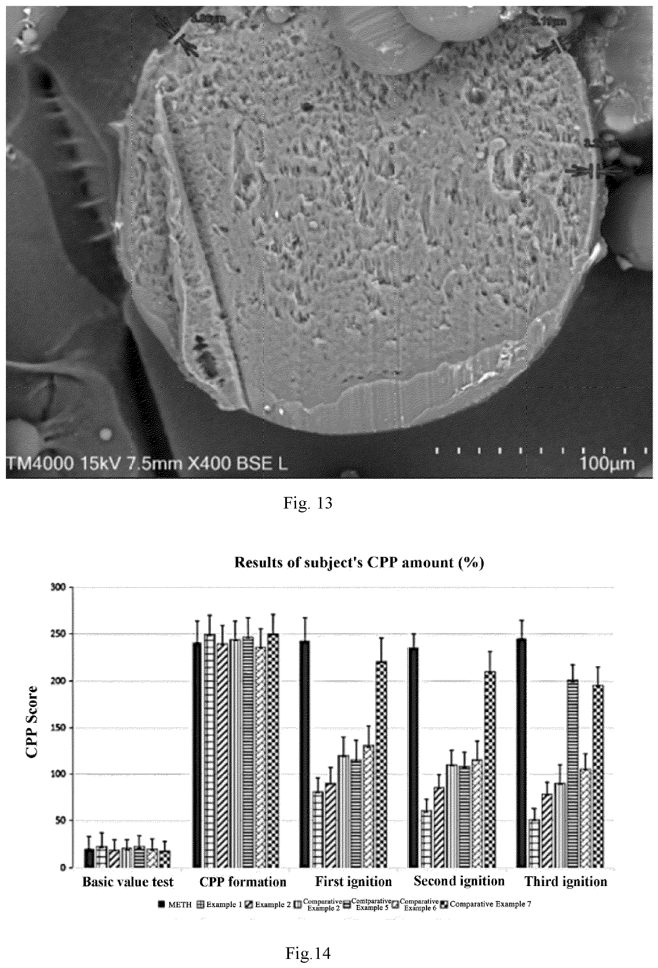

(3) Experimental results were shown in Table 2 and Fig. 14.

**Table 2: Conditioned place preference scores (unit: s)**

| Group | Dose | Basic value test | PCC formation | First ignition | Second ignition | Third ignition |
|---|---|---|---|---|---|---|
| METH | - | 20.3±13. 3 | 241.2±22. 4 | 242.2±25.1 | 235.2±15.1 | 245.5±19.6 |
| Example 1 (20:1) | 3 tablets/ra t | 22.2±14. 2 | 249.5±21. 1 | 80.9±14.4* * | 60.9±12.4* * | 51.7±11.3* |
| Example 2 (30:1) | 3 tablets/ra t | 18.6±10. 8 | 239.3±19. 8 | 90.4±16.6* | 85.4±13.6* | 78.5±12.2* |
| Comparativ e Example 2 (40:1) | 3 tablets/ra t | 20.2±9.8 | 244.1±20. 2 | 120.2±19.6 | 110.1±15.6 * | 90.2±20.1* |
| Comparativ e Example 5 (20:1) | 3 tablets/ra t | 22.0±12. 3 | 246.6±20. 5 | 115.1±21.1 * | 108.6±15.1 * | 200.2±16.7 |
| Comparativ e Example 6 (naltrexone) | 3 tablets/ra t | 19.5±11. 1 | 235.6±19. 8 | 130.5±21.1 | 115.5±20.2 * | 105.6±16.4 * |
| Comparativ e Example 7 (risperidone ) | 3 tablets/ra t | 18.1±9.5 | 250.1±21. 2 | 220.2±25.3 | 210.0±21.4 | 195.2±20.1 |

As can be seen from the data in Table 2, untreated rats and rats in risperidone implant group still had conditioned place preference. After treatment with a specific dose of compound sustained release preparation for naltrexone and naltrexone implant, drug-induced drug-seeking behavior was inhibited, and it was not ignited after 100 days. The results were shown in Table 2 and Fig. 1, there was a significant difference between the different prescription treating groups and the control groups. As can be seen from the comparison of Examples 1 and 2 with Comparative Examples 2, 6 and 7, in a ratio of naltrexone: risperidone = 20-30, naltrexone and risperidone can achieve a synergistic effect of improving methamphetamine addiction symptoms and preventing relapse. In Comparative Example 5, since synchronous release of naltrexone and risperidone was failed, naltrexone was early released, such that the third ignition did not show effect for preventing relapse.

The present application is further elaborated in combination with specific examples. These examples are not used to limit the application, but only to illustrate the application. If the experimental methods used in the examples are not specifically described and the experimental methods with no specific conditions specified in the examples are not specified, they are carried out in accordance with the conventional conditions; unless otherwise specified, the materials, reagents, etc. used in the examples are generally available through commercial means.

## Claims

1. A naltrexone compound sustained-release implant, **characterized by** comprising the following components: naltrexone, risperidone, a degradable material A, a degradable material B, an additive and a lubricant; and a mass ratio of naltrexone to risperidone is 20-30:1.

2. The naltrexone compound sustained-release implant according to claim 1, **characterized in that**
a mass ratio of naltrexone to the degradable material A is (10-40):(60-90);
a mass ratio of risperidone to the degradable material B is (40-60):(40-60);
the degradable material A has a weight-average molecular weight of 10W-15W; and
the degradable material B has a weight-average molecular weight of 2W-4W.

3. The naltrexone compound sustained-release implant according to claim 1, **characterized in that**,
the degradable material A is selected from one or more of the following: PEG-PLGA, PLA, PLGA and PCL;
the degradable material B is selected from one or more of the following: PLA, PLGA and PCL.
the additive is selected from one or more of the following: sodium chloride, sodium bicarbonate, disodium hydrogencarbonate, potassium chloride and phosphate; preferably sodium chloride; and
the lubricant is selected from one or more of the following: magnesium stearate, stearic acid and sodium stearyl fumarate; preferably magnesium stearate.

4. A preparation method of the naltrexone compound sustained-release implant according to any one of claims 1-3, **characterized by** comprising the following steps:
(1) preparation of naltrexone microspheres:
(1.1) dissolving an additive in water, to obtain an aqueous phase W₁;
(1.2) dissolving naltrexone and 30% -60% of a degradable material A in an organic solvent A, to obtain an oil phase O₁;
(1.3) adding the aqueous phase W₁ into the oil phase O₁, ultrasonically emulsifying to form W₁/O₁ primary emulsion;
(1.4) dissolving the remaining degradable material A in the organic solvent A, to obtain an oil phase O₂;
(1.5) adding the W₁/O₁ primary emulsion into the oil phase O₂, ultrasonically emulsifying to form W₁/O₁/O₂ multiple emulsion;
(1.6) preparing an aqueous solution of polyvinyl alcohol (PVA) to obtain an aqueous phase W₂;
(1.7) dispersing the W₁/O₁/O₂ multiple emulsion to the aqueous phase W₂, stirring until the organic solvent A is volatilized, filtering and collecting resulting microspheres, washing and drying to obtain the naltrexone microspheres;
(2) preparation of risperidone microspheres:
(2.1) dissolving risperidone and 50%-80% of a degradable material B in the organic solvent A, to obtain an oil phase O;
(2.2) preparing an aqueous solution of PVA to obtain an aqueous phase W;
(2.3) adding the oil phase O into the aqueous phase W, stirring until the organic solvent A is volatilized, filtering and collecting resulting microspheres, washing and drying to obtain the risperidone microspheres;
(3) preparation of poly lactic acid (PLA) blank particles:
dissolving the remaining degradable material B in an organic solvent B to obtain a solution; adding the solution dropwise into a phosphate buffer to obtain polymer blank particles;
(4) blending and tableting
blending the naltrexone microspheres and the risperidone microspheres, adding and blending with the polymer blank particles and a lubricant, and then pressing into cylindrical tablets;
(5) irradiation sterilization
sterilizing the cylindrical tablets by irradiation to obtain the naltrexone compound sustained-release implant.

5. The preparation method according to claim 4, **characterized in that** in step (1.1), a concentration of the additive in the aqueous phase W₁ is 0-30 mg/mL; preferably 10-25 mg/mL.

6. The preparation method according to claim 4, **characterized in that** in steps (1.2), (1.4), (1.7), (2.1) and (2.3), the organic solvent A is selected from dichloromethane or/and ethyl acetate; preferably dichloromethane.

7. The preparation method according to claim 4, **characterized in that**,
in step (1.2), a concentration of naltrexone in the oil phase O₁ is 100-300 mg/mL; preferably 150-250 mg/mL;
in step (1.2), a concentration of the degradable material A in the oil phase O₁ is 100-300 mg/mL; preferably 100-200 mg/mL; and
in step (1.4), a concentration of the degradable material A in the oil phase O2 is 200-400 mg/mL; preferably 250-350 mg/mL.

8. The preparation method according to claim 4, **characterized in that**,
in steps (1.6) and (2.2), a mass fraction of PVA in the aqueous phase W₂ is 0.1-1.2%; the aqueous phase W₂ also includes sodium chloride, and the sodium chloride has a mass fraction of 0 - 10%;
in step (2.1), a concentration of risperidone in the oil phase O is 50-300 mg/mL; preferably 100-200 mg/mL; a concentration of the degradable material B in the oil phase O is 100-300 mg/mL; preferably 100-200 mg/mL; and
in step (3), a mass fraction of the degradable material B in the solution is 10-25%; preferably 10% - 15%.

9. The preparation method according to claim 4, **characterized in that** in step (4), an amount of the polymer blank particles accounts for 5-10% of the total; an amount of the lubricant accounts for 0-0.05% of the total.

10. Application of the naltrexone compound sustained-release implant according to any one of claims 1-3 in preparation of drugs for treating or relieving amphetamine-type drug addiction.
